# EUROPEAN PATENT SPECIFICATION

(11) **EP 2 123 329 B1**
(45) Date of publication and mention of the grant of the patent: **02.07.2014**
(21) Application number: 09158054.8
(22) Date of filing: 16.04.2009
(51) Int. Cl.: A61Q 5/00, A61K 8/60

(54) **Use of hair care composition**
Verwendung für Haarpflegezusammensetzung
Utilisation de compositions de soins capillaires

(30) Priority: 21.05.2008 EP 08156667
(43) Date of publication of application: 25.11.2009
(73) Proprietor: Unilever PLC, London EC4Y 0DY (GB); Unilever N.V., 3013 AL Rotterdam (NL)
(72) Inventor: Avery, Andrew Richard, Merseyside CH63 3JW (GB); Khoshdel, Ezat, Merseyside CH63 3JW (GB); Ortuoste, Nerea, Merseyside CH63 3JW (GB)
(74) Representative: Tansley, Sally Elizabeth

(56) References cited:
- EP-A- 0 951 898
- WO-A-2005/105031
- WO-A-2007/007291
- GB-A- 1 419 117
- JP-A- 2004 091 399
- JP-A- 2005 330 214
- US-A- 3 519 003

## Description

The present invention relates to the use of a hair care composition for preventing damage to hair.

Hair can suffer damage from a number of sources such as; exposure to UV and chlorine; chemical influences such as bleaching, perming, overly frequent washing with harsh surfactant-based cleansing shampoo compositions; and mechanical influences such as prolonged use of heated styling appliances.

Damage to the hair typically manifests itself in cuticle and protein loss from the hair fibre, hair fibre brittleness and breakage and frayed or split ends.

The present invention has found that the use of a non-surfactant esterified sugar which is sucrose acetate isobutyrate can be used to mitigate damage to hair.

The effect of the invention is particularly enhanced when the compositions of the invention are used as pre-treatment compositions; in that they are applied to the hair, preferably dry hair/towel dried hair and shortly after application (within 1 hour, more preferably within 5 minutes) the hair is washed then rinsed.

Hair is washed in the conventional manner using any commercially available shampoo.

A particularly preferred product for the pre-treatment product is a spray, mousse, gel, cream or lotion. Spray products and/or serums are particularly preferred.

Preferably the non-surfactant esterified sugar is non-crystalline at 20°C.

The level of esterified sugar in the composition is preferably from 0.001 to 10 wt% of the total composition, more preferably from 0.01 to 5 wt%.

It is further preferred if the level of esterified sugar deposited on the hair is from 0.3 to 12 mg of per g of hair, preferably from 0.6 to 6 mg, more preferably form 1 to 1.5 mg.

It is preferred if the non-surfactant esterified sugar is delivered from compositions comprising a solvent for the sugar. Suitable solvent include ethanol, isopropyl alcohol, n-propyl alcohol, n-butyl alcohol isobutyl alcohol, caprylic / capric triglycerides or mixtures thereof. Water may also be present with these solvents. Particularly preferred is ethanol, of particular benefit is an ethanol and water mix.

It is preferred if the weight ratio of sucrose acetate isobutyrate to solvent is from 0.01:100 to 1:100.

Compositions of the invention may further comprise a silicone, more preferably in the form of an emulsion.

In some instances particularly when the product is a serum or oil the compositions may comprise cyclopentasiloxane (D5).

Also preferred for oils/serums are triglycerides.

Amino-functional polydimethyl siloxanes which have the CTFA designation amodimethicone may be used with the present invention. Preferred silicones are emulsions and include silicones such as polydiorganosiloxanes, in particular polydimethylsiloxanes which have the CTFA designation dimethicone, polydimethyl siloxanes having hydroxyl end groups which have the CTFA designation dimethiconol.

Suitable silicone emulsions for use in compositions of the invention are available from suppliers of silicones such as Dow Corning and Momentive. The use of such pre-formed silicone emulsions is preferred for ease of processing and control of silicone particle size. Such pre-formed silicone emulsions will typically additionally comprise a suitable emulsifier such as an anionic or nonionic emulsifier, or mixture thereof, and may be prepared by a chemical emulsification process such as emulsion polymerisation, or by mechanical emulsification using a high shear mixer.

Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions/microemulsions of dimethiconol.

Suitable amodimethicone emulsions are DC939, and SME253 (from Momentive).

Silicone will generally be present in a composition of the invention at levels of from 0.05 to 20%, preferably 1 to 15%, more preferably from 2 to 10% by total weight of silicone based on the total weight of the composition.

In instances where the product is a serum or oil the level of solvent is higher than that stated above, in these cases it is usually from 80 to 99 wt% of the total weight of the composition

The Examples will now be illustrated with reference to the following non-limiting Examples. Inventions according to the invention are demonstrated by a number, comparative inventions are demonstrated by a letter.

### Examples

Hair switches (15cm, 2.5 g) were treated with neat ethanol or 1.5 ml of 0.3 % SAIB (sucrose iosbutyrate) solution in neat ethanol. From these switches 30 hair fibres were extracted.

After drying, these fibres were subjected to 15% strain using a Diastron, at a rate of 200 mm/min and subjected to 5 cycles.

Fibres were suitably prepared and loaded into a scanning electron microscope and imaged under 500 times magnification. The operator sampled three locations at random on each switch and judged the level of damage from the image.

| Damage Type | Control 100% ethanol | Example1 0.3 wt% SAIB, 99.7 wt% ethanol |
|---|---|---|
| Not measurable | 4 | 7 |
| Virgin like appearance | 15 | 37 |
| Low level of cuticle damage | 36 | 29 |
| Medium Level of cuticle damage | 23 | 15 |
| High level of cuticle damage | 12 | 2 |

Hair which had been exposed to the example according to the invention showed more regions of virgin like appearance and fewer regions of damaged appearance than did the control samples. This shows that hair treated with SAIB is not so easily damaged as untreated hair.

**A cream Composition**

| INCI Name | Supplier | % active in raw material | Invention Example 2 |
|---|---|---|---|
| dimethicone and amodimethicone | Dow Corning | 70 | 2.69 |
| glycerol | BDH | 100 | 1.60 |
| behenyl alcohol | Cognis | 100 | 2.00 |
| stearamidopropyl dimethylamine | Inolex | 100 | 0.80 |
| hydroxyethyl cellulose | Aqualon | 100 | 0.24 |
| lactic acid | Purac | 88 | 0.16 |
| sucrose acetate isobutyrate (SAIB) and ethanol | Eastman | 90 | 0.33 |
| mineral oil | Silkolene | 100 | 4.50 |
| water and minors | | 100 | To 100 |

**A serum formulation**

| chemical name | % in raw material | % w/w Example 3 water in oil serum |
|---|---|---|
| alpha,omega dihydroxylated polydimethylsiloxane / polydimethylsiloxane | 12 | 23.10 |
| hydroxyethyl cellulose | 100 | 0.21 |
| PEG-400 | 100 | 3.50 |
| Propylene glycol | 100 | 41.57 |
| Sodium Chloride | 100 | 1.40 |

## Claims

1. Use of a non-surfactant esterified sugar which is sucrose acetate isobutyrate to mitigate damage to hair.

2. Use according to any preceding claim in which the level of esterified sugar in a composition used to treat hair is from 0.01 to 5 wt% of the total composition.

3. Use according to any preceding claim in which the esterified sugar is in a composition in the form of a spray.

4. Use according to any preceding claim in which the esterified sugar is in a composition further comprising ethanol.

5. Use according to claim 4 in which the composition further comprises a silicone.

## Patentansprüche

1. Verwendung eines kein Tensid darstellenden, veresterten Zuckers, der Saccharoseacetatisobutyrat ist, um eine Haarschädigung zu mildern.

2. Verwendung nach einem der vorstehenden Ansprüche,
wobei die Menge an verestertem Zucker in einer für die Haarbehandlung verwendeten Zusammensetzung 0,01 bis 5 Gew.-% der gesamten Zusammensetzung beträgt.

3. Verwendung nach einem der vorstehenden Ansprüche,
wobei der veresterte Zucker in einer Zusammensetzung in Form eines Sprays vorliegt.

4. Verwendung nach einem der vorstehenden Ansprüche,
wobei der veresterte Zucker in einer Zusammensetzung vorliegt, die ferner Ethanol aufweist.

5. Verwendung nach Anspruch 4,
wobei die Zusammensetzung ferner ein Silicon aufweist.

## Revendications

1. Utilisation d'un sucre estérifié non tensio-actif qui est l'acétate isobutyrate de saccharose pour atténuer les dégâts occasionnés sur la chevelure.

2. Utilisation selon l'une quelconque des revendications précédentes, dans laquelle le taux de sucre estérifié dans une composition employée pour traiter la chevelure représente de 0,01 à 5 % en poids de la composition totale.

3. Utilisation selon l'une quelconque des revendications précédentes, dans laquelle le sucre estérifié est contenu dans une composition sous forme de spray.

4. Utilisation selon l'une quelconque des revendications précédentes, dans laquelle le sucre estérifié est contenu dans une composition comprenant en outre de l'éthanol.

5. Utilisation selon la revendication 4, dans laquelle la composition comprend en outre une silicone.
